# EUROPEAN PATENT APPLICATION

(11) **EP 4 374 885 A1**
(43) Date of publication of application: **29.05.2024**
(21) Application number: 22846254.5
(22) Date of filing: 21.07.2022
(51) Int. Cl.: A61L 27/18, A61F 2/00, C08G 63/664, C08G 65/332

(54) **POWDER FORMULATION FOR TISSUE REPAIR, PREPARATION METHOD THEREFOR, AND INJECTABLE COMPOSITION FOR TISSUE REPAIR, COMPRISING SAME**

(30) Priority: 21.07.2021 KR 20210095722
(71) Applicant: Samyang Holdings Corporation, Seoul 03129 (KR)
(72) Inventor: PARK, Ji Hoon, Suwon-si Gyeonggi-do 16509 (KR); YOON, Hye Sung, Seongnam-si Gyeonggi-do 13544 (KR); LIM, Soo Mee, Seongnam-si Gyeonggi-do 13439 (KR)
(74) Representative: Dietz, Christopher Friedrich
(86) International application number: PCT/KR2022/010688
(87) International publication number: WO 2023/003384

(57) **Abstract**

The present invention relates to a powder formulation for tissue repair, a preparation method therefor, and an injectable composition for tissue repair, comprising same, and, more specifically, to: a powder formulation, a preparation method therefor, and an injectable composition for tissue repair, comprising same, the powder formulation comprising a biocompatible copolymer, which is a copolymer of a hydrophobic biocompatible polymer and a hydrophilic biocompatible polymer and forms, in an aqueous medium, micelle-type nanoparticles with an average diameter of a specific range and/or has a ratio, of a specific level or less, of the hydrophilic biocompatible polymer to the hydrophobic biocompatible polymer, being in a powder form so as to facilitate transport, storage and handling, being readily dissolved in an aqueous medium at room temperature so as to form stable nanoparticles, and allowing collagen induction to be performed without being phagocytosed by macrophages after being introduced into the body, thereby exhibiting an excellent tissue repair effect.

## Description

### TECHNICAL FIELD

The present invention relates to a powder formulation for tissue repair treatment and a method for preparing the same, and an injection composition for tissue repair treatment comprising the same, and more specifically, a powder formulation comprising a copolymer of hydrophilic biocompatible polymer and hydrophobic biocompatible polymer, which is a biocompatible copolymer forming nanoparticles in micelle form with an average diameter within a specific range in an aqueous medium and/or having the ratio of the molecular weight of the hydrophilic polymer to the molecular weight of the hydrophobic polymer below a specific level, whereby the formulation in powder form is easily transported, stored and handled, and easily dissolved in aqueous medium at room temperature to form stable nanoparticles, and after being introduced into the body, it is not phagocytosed by macrophages and induces collagen, showing excellent tissue repair effect, and a method for preparing the same and an injection composition for tissue repair treatment comprising the same.

### BACKGROUND ART

Recently, many people are interested in well-aging, which is growing old gracefully from a young age. It is no exaggeration to say that the current beauty market is focusing on anti-aging, which is aging slowly, beautifully and healthily. One of the most common signs of aging is a decrease in volume. In particular, since no volume in the face can make older and shabby look, people are very interested in fillers that can provide volume. Accordingly, the filler market is growing rapidly every year, and currently forms a market worth more than 2 trillion won worldwide.

Various substances are currently used as filler materials, and among them hyaluronic acid fillers occupy more than 90% of the global filler market, but have problems that the half-life in the body is within 1 to 3 days and so the persistence is very low and the resorption in the body occurs very rapidly. Thus, products having a resorption period extended by connecting hyaluronic acid with crosslinking material are on market. However, in case of such crosslinked products, because the crosslinking material, BDDE (1,4-butanediol diglycidyl ether), is a toxic carcinogen, there are problems that process cost increases according to removal process thereof, and loss is caused by product disposal due to microbial contamination, product disposal due to detection of residues, etc.

Accordingly, many tissue repair products using polymers that decompose in vivo have been developed, and as filler formulations using conventional biocompatible polymers, formulations have been developed by processing water-insoluble polymers into micro-sized particles and then dispersing them through a viscous excipient or thickener, and used. For example, a formulation in which poly(lactic acid) (PLA) particles with a diameter of 20 to 50 micrometers are dispersed in an aqueous solution of carboxymethylcellulose (CMC) or a formulation in which poly(caprolactone) (PCL) particles with a diameter of 20 to 50 micrometers are dispersed in an aqueous solution of CMC and glycerin have been used, but such formulations have problems of inconvenience in surgery due to needle clogging by the microparticles during injection and failure to obtain uniform tissue repair effect due to not uniformly dispersed particles.

### CONTENTS OF THE INVENTION

### PROBLEMS TO BE SOLVED

The purpose of the present invention is to provide a tissue repair product which has no risk of toxicity, secures functionality, properties, and safety suitable for biomaterials for tissue repair, and exhibits excellent tissue repair effects while being easily transported, stored, and handled, and a method for preparing the same.

### TECHNICAL MEANS

In an aspect, the present invention provides a powder formulation for tissue repair treatment, comprising a biocompatible copolymer which is a copolymer of hydrophilic biocompatible polymer and hydrophobic biocompatible polymer, wherein:
(1) the biocompatible copolymer forms nanoparticles in micelle form in an aqueous medium with an average diameter of from greater than 20 nm to 100 nm or less as measured by dynamic light scattering method [condition (1)]; or
(2) in the biocompatible copolymer, the ratio (Mn1/Mn2) of the number average molecular weight of the hydrophilic biocompatible polymer (Mn1) to the number average molecular weight of the hydrophobic biocompatible polymer (Mn2) is 1.84 or less [condition (2)]; or
(3) the biocompatible copolymer satisfies all of the above conditions (1) and (2).

In other aspect, the present invention provides a method for preparing a powder formulation for tissue repair treatment, the method comprising: polymerizing monomers for hydrophobic biocompatible polymer in the presence of hydrophilic biocompatible polymer to prepare a biocompatible copolymer which is a copolymer of hydrophilic biocompatible polymer and a hydrophobic biocompatible polymer; and drying the prepared copolymer; wherein:
(1) the biocompatible copolymer forms nanoparticles in micelle form in an aqueous medium with an average diameter of from greater than 20 nm to 100 nm or less as measured by dynamic light scattering method [condition (1)]; or
(2) in the biocompatible copolymer, the ratio (Mn1/Mn2) of the number average molecular weight of the hydrophilic biocompatible polymer (Mn1) to the number average molecular weight of the hydrophobic biocompatible polymer (Mn2) is 1.84 or less [condition (2)]; or
(3) the biocompatible copolymer satisfies all of the above conditions (1) and (2).

In another aspect, the present invention provides an injection composition for tissue repair treatment, comprising the powder formulation for tissue repair treatment of the present invention; and a pharmaceutically acceptable carrier for injection.

In still another aspect, the present invention provides a method for preparing an injection composition for tissue repair treatment, the method comprising: mixing the powder formulation for tissue repair treatment of the present invention and a pharmaceutically acceptable carrier for injection at room temperature.

### EFFECT OF THE INVENTION

The powder formulation for tissue repair treatment of the present invention has no toxicity and is easily transported, stored and handled, and easily dissolved in aqueous medium at room temperature to form stable nanoparticles in micelle form in an aqueous medium with an average diameter of from greater than 20 nm to 100 nm or less, and after being introduced into the body, the particles undergo self-assembly through hydrophobic aggregation of hydrophobic polymer under the influence of the environment in the body to form a large structure, which is not phagocytosed by macrophages and induces collagen, resulting in exhibition of excellent tissue repair effect.

### BRIEF EXPLANATION OF THE DRAWINGS

Figure 1 shows the results of measuring the particle size conducted in Experimental Example 2 of the present invention.
Figure 2 shows the results of animal tests for tissue repair effect conducted in Experimental Example 4 of the present invention.

### CONCRETE MODE FOR CARRYING OUT THE INVENTION

The present invention will be explained in detail below.

The powder formulation for tissue repair treatment of the present invention comprises a biocompatible copolymer which is a copolymer of hydrophilic biocompatible polymer and hydrophobic biocompatible polymer.

In an embodiment, the biocompatible copolymer forms nanoparticles in micelle form in an aqueous medium with an average diameter of from greater than 20 nm to 100 nm or less as measured by dynamic light scattering method [**condition (1)**].

The aqueous medium may be, for example, water (more concretely, distilled water).

The dynamic light scattering (DLS) method, which is a non-invasive and well-established technique to measure the size of nanoparticles in a dispersion conventionally, measures the intensity of scattered light over time in a suspension using the Brownian motion of particles. The variation in such intensity of scattered light is converted into a diffusion coefficient for determining an average diameter of particles, and through the Stokes-Einstein relationship, the average diameter of the particles can be determined from the diffusion coefficient.

If the average diameter of the nanoparticles (as measured by DLS) formed by the biocompatible copolymer in an aqueous medium is 20 nm or less or greater than 100 nm, a powder formulation prepared using such a copolymer may not exhibit tissue repair effect.

More concretely, the average diameter of the nanoparticles (as measured by DLS) formed by the biocompatible copolymer in an aqueous medium may be greater than 20 nm, 21 nm or greater, 25 nm or greater, 30 nm or greater, 35 nm or greater, 40 nm or greater, 45 nm or greater, 50 nm or greater, or 55 nm or greater, and also it may be 100 nm or less, less than 100 nm, 95 nm or less, 90 nm or less, or 85 nm or less, but it is not limited thereto.

In other embodiment, in the biocompatible copolymer, the ratio (Mn1/Mn2) of the number average molecular weight of the hydrophilic biocompatible polymer (Mn1) to the number average molecular weight of the hydrophobic biocompatible polymer (Mn2) is 1.84 or less [**condition (2)**].

The number average molecular weights of the hydrophilic and hydrophobic biocompatible polymers may be measured, for example, by gel permeation chromatography (GPC).

Gel permeation chromatography (GPC) is a method wherein the physical elution mechanism is that large components are eluted first and small components are eluted later depending on the size of the hydrodynamic volume of the analyzed components, and thus large molecules cannot enter the pores of the porous gel and pass through quickly while small molecules can enter the pores of the gel and be retained therein and thus pass through slowly, and the relative molecular weights are analyzed in the order of molecules passing through the column more quickly.

If the Mn1/Mn2 ratio in the biocompatible copolymer is greater than 1.84, a powder formulation prepared using such a copolymer may not exhibit tissue repair effect.

More concretely, the Mn1/Mn2 ratio in the biocompatible copolymer may be 1.84 or less, 1.82 or less, 1.8 or less, 1.78 or less, 1.76 or less, or 1.75 or less. There is no special limitation to the lower limit of the Mn1/Mn2 ratio, and for example, it may be 0.5 or more, 0.55 or more, 0.6 or more, 0.65 or more, 0.7 or more, 0.75 or more, 0.8 or more, 0.85 or more, 0.9 or more, or 0.95 or more, but it is not limited thereto.

In a preferable embodiment, the biocompatible copolymer satisfies all of the above conditions (1) and (2).

Concretely, the hydrophilic biocompatible polymer may be selected from the group consisting of polyethylene glycol or its derivative (e.g., alkoxy- or hydroxy-polyethylene glycol), polyvinylpyrrolidone, polyvinyl alcohol, polyacrylamide, and combination thereof, and more concretely, it may be selected from the group consisting of polyethylene glycol (PEG), methoxypolyethylene glycol (mPEG), and combination thereof.

Also concretely, the hydrophobic biocompatible polymer may be polymer of alpha (α)-hydroxy acid-derived monomers, and more concretely, it may be selected from the group consisting of polylactide, polyglycolide, poly(lactic-glycolide), polymandelic acid, polycaprolactone, polydioxan-2-one, polyamino acid, polyorthoester, polyanhydride, polycarbonate and combination thereof, and still more concretely, it may be selected from the group consisting of polylactide, polyglycolide, poly(lactic-glycolide) and combination thereof.

Concretely, the number average molecular weight (unit: g/mol) by GPC of the hydrophilic biocompatible polymer (Mn1) may be 1,000 or more, 2,000 or more, 3,000 or more, or 4,000 or more, and also it may be 30,000 or less, 25,000 or less, 20,000 or less, or 15,000 or less.

Concretely, the number average molecular weight (unit: g/mol) by GPC of the hydrophobic biocompatible polymer (Mn2) may be 500 or more, 1,000 or more, 1,500 or more, or 2,000 or more, and also it may be 30,000 or less, 25,000 or less, 20,000 or less, or 15,000 or less.

Concretely, the total number average molecular weight by GPC of the biocompatible copolymer may be 2,000 or more, 5,000 or more, 8,000 or more, or 10,000 or more, and also it may be 40,000 or less, 35,000 or less, 30,000 or less, or 25,000 or less.

In addition to the biocompatible copolymer explained above, the powder formulation for tissue repair treatment of the present invention may further comprise one or more additives that can be conventionally used in pharmaceutical powder formulation.

In an embodiment, the powder formulation for tissue repair treatment of the present invention may further comprise freeze-drying aid (also called freeze-drying agent).

Concretely, the freeze-drying aid may be one or more selected from the group consisting of lactose, maltose, sucrose, trehalose, mannitol, sorbitol, maltitol, xylitol, and lactitol.

In an embodiment, the powder formulation for tissue repair treatment of the present invention may further comprise local anesthetic.

Concretely, the local anesthetic may be one or more selected from the group consisting of ambucaine, amolanone, amylocaine, benoxinate, benzocaine, betoxycaine, biphenamine, bupivacaine, butacaine, butamben, butanilicaine, butethamine, butoxycaine, carticaine, chloroprocaine, cocaethylene, cocaine, cyclomethycaine, dibucaine, dimethysoquin, dimethocaine, diperodon, dicyclonine, ecgonidine, ecgonine, ethyl chloride, etidocaine, beta-eucaine, euprocin, fenalcomine, formocaine, hexylcaine, hydroxytetracaine, isobutyl p-aminobenzoate, leucinocaine mesylate, levoxadrol, lidocaine, mepivacaine, meprylcaine, metabutoxycaine, methyl chloride, myrtecaine, naepaine, octacaine, orthocaine, oxethazaine, parethoxycaine, phenacaine, phenol, piperocaine, piridocaine, polidocanol, pramoxine, prilocaine, procaine, propanocaine, proparacaine, propipocaine, propoxycaine, pseudococaine, pyrrocaine, ropivacaine, salicyl alcohol, tetracaine, tolycaine, trimecaine, zolamine, and salts thereof.

The powder formulation for tissue repair treatment of the present invention may be a formulation dried by freeze-drying or other drying method (e.g., spin drying, etc.) and thus having a powder status.

The other aspect of the present invention provides a method for preparing a powder formulation for tissue repair treatment, the method comprising: polymerizing monomers for hydrophobic biocompatible polymer in the presence of hydrophilic biocompatible polymer to prepare a biocompatible copolymer which is a copolymer of hydrophilic biocompatible polymer and a hydrophobic biocompatible polymer; and drying the prepared copolymer; wherein: (1) the biocompatible copolymer forms nanoparticles in micelle form in an aqueous medium with an average diameter of from greater than 20 nm to 100 nm or less as measured by dynamic light scattering method; or (2) in the biocompatible copolymer, the ratio (Mn1/Mn2) of the number average molecular weight of the hydrophilic biocompatible polymer (Mn1) to the number average molecular weight of the hydrophobic biocompatible polymer (Mn2) is 1.84 or less; or (3) the biocompatible copolymer satisfies all of the above conditions (1) and (2).

In the method for preparing a powder formulation for tissue repair treatment of the present invention, the hydrophilic biocompatible polymer, hydrophobic biocompatible polymer and biocompatible copolymer are the same as explained above.

Concretely, the monomer for hydrophobic biocompatible polymer may be alpha (α)-hydroxy acid-derived monomer, and more concretely, it may be selected from the group consisting of lactide, glycolide, mandelic acid, caprolactone and combination thereof, and still more concretely, it may be selected from the group consisting of lactide, glycolide, mandelic acid and combination thereof, but it is not limited thereto.

The step of polymerizing monomers for the hydrophobic biocompatible polymer in the presence of the hydrophilic biocompatible polymer can be conducted according to known method and condition.

Concretely, the drying of the biocompatible copolymer can be conducted by freeze-drying or other drying method (e.g., spin drying, etc.), and more concretely it can be conducted by freeze-drying, but it is not limited thereto. The freeze-drying of the biocompatible copolymer can be conducted in the presence of freeze-drying aid as explained above, but it is not limited thereto. The powder formulation for tissue repair treatment of the present invention is easily dissolved in aqueous medium at room temperature to form stable nanoparticles in micelle form in an aqueous medium with an average diameter of from greater than 20 nm to 100 nm or less, and after being introduced into the body, the particles undergo self-assembly through hydrophobic aggregation of hydrophobic polymer under the influence of the environment in the body to form a large structure, which is not phagocytosed by macrophages and induces collagen, resulting in exhibition of excellent tissue repair effect.

In the present invention, the tissue repair effect refers to the effect of returning the tissue to its original state when necrosis, defects, etc. occur in skin tissue due to trauma, inflammation, aging, etc.

Therefore, another aspect of the present invention provides an injection composition for tissue repair treatment, comprising the powder formulation for tissue repair treatment of the present invention; and a pharmaceutically acceptable carrier for injection.

As the pharmaceutically acceptable carrier for injection comprised in the injection composition for tissue repair treatment of the present invention, conventional one can be used without limitation, and for example, it may be selected from the group consisting of distilled water for injection, physiological saline, 5% glucose, buffer solutions (e.g., phosphate buffer solution (PBS)), and combination thereof, but it is not limited thereto.

In addition to the components explained above, the injection composition for tissue repair treatment of the present invention may further comprise one or more conventional additives that can be used in injection formulation.

Still another aspect of the present invention provides a method for preparing an injection composition for tissue repair treatment, the method comprising: mixing the powder formulation for tissue repair treatment of the present invention and a pharmaceutically acceptable carrier for injection at room temperature.

In the method for preparing an injection composition for tissue repair treatment of the present invention, "room temperature" means 1 to 30°C, 20 to 30°C, 22 to 28°C, more specifically 24 to 26°C (e.g., 25°C).

The powder formulation of the existing tissue repair polymer product must be heated (e.g., the temperature must be raised between the melting point of the polymer and the boiling point of water) to be manufactured in the form of an aqueous solution. However, the powder formulation for tissue repair treatment of the present invention is easily dissolved in aqueous medium even at room temperature, and thus its injectable composition in the form of an aqueous solution can be easily prepared and used at room temperature.

The present invention will be explained below in more detail with reference to the following Examples. However, the Examples are only to illustrate the invention, and the scope of the present invention is not limited thereby in any manner.

### EXAMPLES

### Preparation Example 1: Preparation of powder formulation

In order to obtain a biocompatible copolymer with a target number average molecular weight (Mn) of 15,000 g/mol, in the presence of methoxypolyethyleneglycol (mPEG) with a number average molecular weight (Mn1) of 9,850 g/mol (measured by GPC) as a hydrophilic biocompatible polymer, D,L-lactide monomer was polymerized in the presence of a catalyst, and then the resulting copolymer was dried to obtain a powder formulation. As a result of GPC measurement, the number average molecular weight (Mn) of the prepared mPEG-Poly(D,L-lactide) copolymer was 15,500 g/mol, and the hydrophobic polymer molecular weight was calculated by subtracting the hydrophilic polymer molecular weight from the measured molecular weight of the copolymer. The ratio (Mn1/Mn2) of the number average molecular weight (Mn1) of mPEG (hydrophilic polymer in the copolymer) to the number average molecular weight (Mn2) of Poly(D,L-lactide) (hydrophobic polymer in the copolymer) was 1.74.

### Preparation Example 2: Preparation of powder formulation

Excepting that the target number average molecular weight (Mn) of the biocompatible copolymer was set to 17,000 g/mol, a powder formulation was prepared in the same manner as Preparation Example 1. As a result of GPC measurement, the number average molecular weight (Mn) of the prepared mPEG-Poly(D,L-lactide) copolymer was 17,200 g/mol, and the hydrophobic polymer molecular weight was calculated by subtracting the hydrophilic polymer molecular weight from the measured molecular weight of the copolymer. The ratio (Mn1/Mn2) of the number average molecular weight (Mn1) of mPEG (hydrophilic polymer in the copolymer) to the number average molecular weight (Mn2) of Poly(D,L-lactide) (hydrophobic polymer in the copolymer) was 1.34.

### Preparation Example 3: Preparation of powder formulation

Excepting that the target number average molecular weight (Mn) of the biocompatible copolymer was set to 20,000 g/mol, a powder formulation was prepared in the same manner as Preparation Example 1. As a result of GPC measurement, the number average molecular weight (Mn) of the prepared mPEG-Poly(D,L-lactide) copolymer was 20,200 g/mol, and the hydrophobic polymer molecular weight was calculated by subtracting the hydrophilic polymer molecular weight from the measured molecular weight of the copolymer. The ratio (Mn1/Mn2) of the number average molecular weight (Mn1) of mPEG (hydrophilic polymer in the copolymer) to the number average molecular weight (Mn2) of Poly(D,L-lactide) (hydrophobic polymer in the copolymer) was 0.95.

### Comparative Example 1

A formulation prepared as powder by freeze-drying a commercial product consisting of mPEG-Polycaprolactone copolymer (Miracle L, Dexlevo Co., Ltd.) was used as Comparative Example 1.

### Comparative Example 2: Preparation of powder formulation

Excepting that the target number average molecular weight (Mn) of the biocompatible copolymer was set to 12,000 g/mol, a powder formulation was prepared in the same manner as Preparation Example 1. As a result of GPC measurement, the number average molecular weight (Mn) of the prepared mPEG-Poly(D,L-lactide) copolymer was 12,350 g/mol, and the hydrophobic polymer molecular weight was calculated by subtracting the hydrophilic polymer molecular weight from the measured molecular weight of the copolymer. The ratio (Mn1/Mn2) of the number average molecular weight (Mn1) of mPEG (hydrophilic polymer in the copolymer) to the number average molecular weight (Mn2) of Poly(D,L-lactide) (hydrophobic polymer in the copolymer) was 2.8.

### Comparative Example 3: Preparation of powder formulation

In order to obtain a biocompatible copolymer with a target number average molecular weight (Mn) of 25,000 g/mol, in the presence of methoxypolyethyleneglycol (mPEG) with a number average molecular weight (Mn1) of 20,000 g/mol (measured by GPC) as a hydrophilic biocompatible polymer, D,L-lactide monomer was polymerized in the presence of a catalyst, and then the resulting copolymer was dried to obtain a powder formulation. As a result of GPC measurement, the number average molecular weight (Mn) of the prepared mPEG-Poly(D,L-lactide) copolymer was 25,650 g/mol, and the hydrophobic polymer molecular weight was calculated by subtracting the hydrophilic polymer molecular weight from the measured molecular weight of the copolymer. The ratio (Mn1/Mn2) of the number average molecular weight (Mn1) of mPEG (hydrophilic polymer in the copolymer) to the number average molecular weight (Mn2) of Poly(D,L-lactide) (hydrophobic polymer in the copolymer) was 3.5.

### Comparative Example 4: Preparation of powder formulation

In order to obtain a biocompatible copolymer with a target number average molecular weight (Mn) of 23,000 g/mol, in the presence of methoxypolyethyleneglycol (mPEG) with a number average molecular weight (Mn1) of 20,000 g/mol (measured by GPC) as a hydrophilic biocompatible polymer, D,L-lactide monomer was polymerized in the presence of a catalyst, and then the resulting copolymer was dried to obtain a powder formulation. As a result of GPC measurement, the number average molecular weight (Mn) of the prepared mPEG-Poly(D,L-lactide) copolymer was 23,500 g/mol, and the hydrophobic polymer molecular weight was calculated by subtracting the hydrophilic polymer molecular weight from the measured molecular weight of the copolymer. The ratio (Mn1/Mn2) of the number average molecular weight (Mn1) of mPEG (hydrophilic polymer in the copolymer) to the number average molecular weight (Mn2) of Poly(D,L-lactide) (hydrophobic polymer in the copolymer) was 5.7.

### Comparative Example 5: Preparation of powder formulation

In order to obtain a biocompatible copolymer with a target number average molecular weight (Mn) of 15,000 g/mol, in the presence of methoxypolyethyleneglycol (mPEG) with a number average molecular weight (Mn1) of 9,800 g/mol (measured by GPC) as a hydrophilic biocompatible polymer, ε-caprolactone monomer was polymerized in the presence of a catalyst, and then the resulting copolymer was dried to obtain a powder formulation. As a result of GPC measurement, the number average molecular weight (Mn) of the prepared mPEG-PCL copolymer was 15,100 g/mol, and the hydrophobic polymer molecular weight was calculated by subtracting the hydrophilic polymer molecular weight from the measured molecular weight of the copolymer. The ratio (Mn1/Mn2) of the number average molecular weight (Mn1) of mPEG (hydrophilic polymer in the copolymer) to the number average molecular weight (Mn2) of the hydrophobic polymer in the copolymer was 1.85.

When the mPEG-PCL was prepared as an aqueous solution by mixing at room temperature, particle size analysis was difficult since the polymer particles were precipitated due to poor dispersion stability, and the reliability of the results was low. Therefore, water was added to the mPEG-PCL, and the mixture was heated to 80°C and then mixed to prepare an aqueous polymer colloidal solution, and the solution was diluted to 5 % by weight concentration and then particle size analysis was conducted thereto.

### Experimental Example 1: Analysis of polymer molecular weight

The number average molecular weights (Mn) of the copolymers of Preparation Examples 1 to 3 and Comparative Examples 1 to 5 were measured by performing Gel Permeation Chromatography (GPC) under the conditions shown in the following table. The results are shown in Table 1 below.

| Instrument | Agilent Infinity 1260 GPC/SEC system |
|---|---|
| Column | One Mixed-C guard column and two Mixed-C analytical columns |
| Eluent | Chloroform (HPLC grade) |
| Detector | Refractive Index (RI) Detector |
| Flow rate | 1.0 mL/min |
| Temperature | 35°C |
| Concentration | 0.1∼ 0.2 w/v % |
| Injection volume | 100 µL |
| Standard material | 9 kinds of PS (195300/69650/30230/19500/12980/6320/3090/1290/945g/mol) |

### Experimental Example 2: Measurement of the average diameter of polymer nanoparticles in aqueous medium

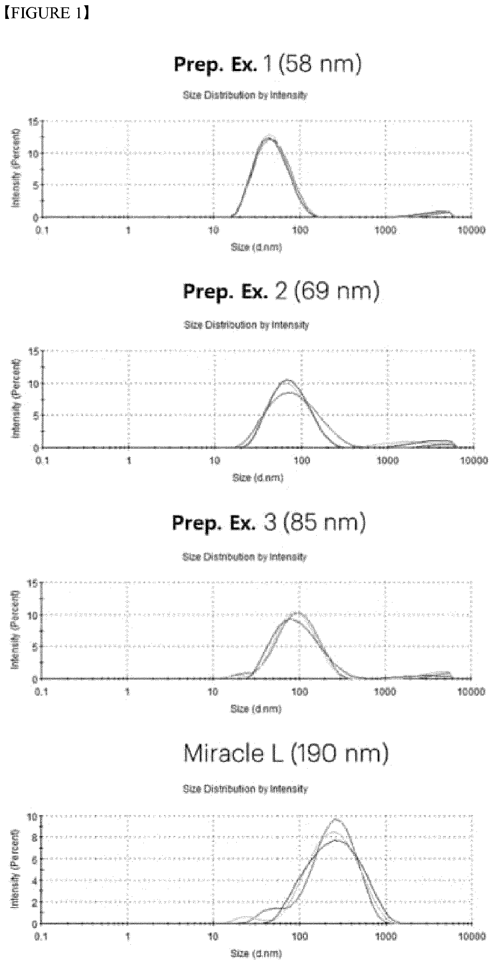

Each of the formulations of Preparation Examples 1 to 3 and Comparative Examples 1 to 5 was added to water for injection as aqueous medium to prepare a solution with concentration of 5 % by weight, and the average diameter of polymer nanoparticles formed in the aqueous medium was measured by Dynamic Light Scattering (DLS) method using Zetasizer Nano Zs90 of Malvern Co., Ltd. When dissolving the powder formulations of Preparation Examples 1 to 3 in the aqueous medium, it was confirmed that polymer nanoparticles of a fine size, which were difficult to see with the naked eye, were formed in the aqueous medium. The results of measurement of the average diameter are shown in Figure 1 and the following Table 1.

**[Table 1]**

| | Hydrophilic polymer M.W. (Mn1) | Hydrophobic polymer M.W. (Mn2) | Copolymer M.W. | Mn1/Mn2 ratio | Average diameter (nm) |
|---|---|---|---|---|---|
| Prep. Ex. 1 | 9,850 | 5,650 | 15,500 | 1.74 | 58 |
| Prep. Ex. 2 | 9,850 | 7,350 | 17,200 | 1.34 | 69 |
| Prep. Ex. 3 | 9,850 | 10,350 | 20,200 | 0.95 | 85 |
| Comp. Ex. 1 (Miracle L) | - | - | 25,300 | - | 190 |
| Comp. Ex. 2 | 9,850 | 3,500 | 12,350 | 2.8 | 19 |
| Comp. Ex. 3 | 20,000 | 5,650 | 25,650 | 3.5 | 120 |
| Comp. Ex. 4 | 20,000 | 3,500 | 23,500 | 5.7 | 20 |
| Comp. Ex. 5 (mPEG-PCL) | 9,850 | 5,300 | 15,150 | 1.85 | 69 |

| | | | | | |
|---|---|---|---|---|---|
| (Prep. Ex.: Preparation Example; Comp. Ex.: Comparative Example; M.W.: Molecular weight) | | | | | |

### Experimental Example 3: Confirmation about stability of the aqueous solution of powder formulation

The powder formulation of Preparation Example 3 was dissolved in water for injection at a concentration of 5 % by weight at room temperature, and the stability of the aqueous solution was confirmed by observing the state over time. The powder formulation of Preparation Example 3 was completely dissolved within 20 minutes and no particles were observed with the naked eye, and even after being left for 3 hours, without significant change, no particles were observed with the naked eye.

For comparison, the freeze-dried powder formulation of the Comparative Example product (Miracle L) was added to water for injection at a concentration of 5 % by weight at room temperature, and the state over time was observed. The dispersion of the freeze-dried powder formulation of the Comparative Example product (Miracle L) was opaque and cloudy, and even after 3 hours, the particles were not dissolved but precipitated or dispersed in water for injection.

### Experimental Example 4: Verification of efficacy as a formulation for tissue repair through animal tests

The efficacy for tissue repair of the powder formulation according to the present invention was verified through animal tests. The animal tests were conducted using 5-week-old SD rats (purchased from Orient Bio Co., Ltd.).

In the animal tests, saline and test materials were administered to each 5-week-old SD rat on both sides. During the test period, the rearing environment was set at a temperature of 24 ± 2°C, relative humidity of 50 ± 10%, and lighting time of 12 hours, and food was allowed to be eaten freely.

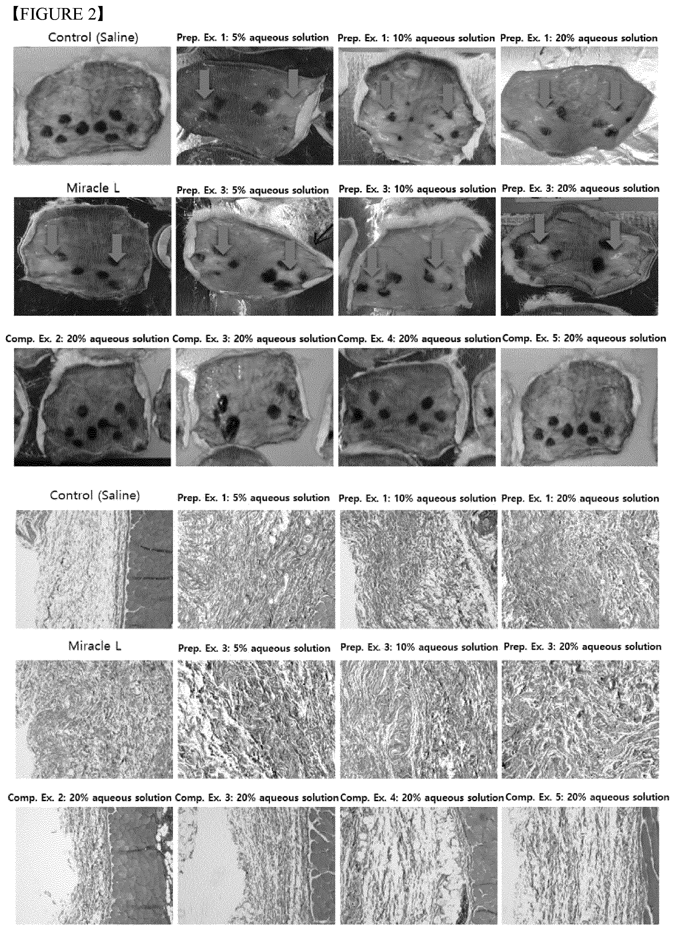

Physiological saline as Control, an aqueous solution of each powder formulation prepared in Preparation Examples 1 to 3 dissolved in water for injection (5 %, 10 %, 20% concentrations) as a test material, and Comparative Example 1 product (Miracle L) and an aqueous solution of each powder formulation of Comparative Examples 2 to 5 dissolved in water for injection (20 % concentration) as comparison group were injected at a constant rate of 100 µl. After 6 weeks, the test animals were sacrificed, and the skin tissue at the sample injection site (the area indicated by the arrow in the upper part of Figure 2) was stained with Masson's Trichorme (MT) and collagen formation in the tissue was to observed in order to evaluate the ability of new collagen biosynthesis. The results are shown in Figure 2 (the lower part of Figure 2).

From Figure 2, it was confirmed that, as compared with Comparative Examples, especially Miracle L, the aqueous solutions of the formulations of Preparation Examples showed excellent collagen formation, and in particular, the formulation of Preparation Example 3 had a large number of formed collagen fibers which were significantly thicker than Comparative Examples, especially Miracle L.

That is, it was thereby confirmed that as compared with Comparative Examples, especially Miracle L, the powder formulation according to the present invention has the advantage that it is easily transported, stored and handled, and easily dissolved in aqueous medium at room temperature, and after being introduced into the body, it induces collagen formation more efficiently, and thus shows excellent tissue repair effect.

## Claims

1. A powder formulation for tissue repair treatment, comprising a biocompatible copolymer which is a copolymer of hydrophilic biocompatible polymer and hydrophobic biocompatible polymer, wherein:
(1) the biocompatible copolymer forms nanoparticles in micelle form in an aqueous medium with an average diameter of from greater than 20 nm to 100 nm or less as measured by dynamic light scattering method [condition (1)]; or
(2) in the biocompatible copolymer, the ratio (Mn1/Mn2) of the number average molecular weight of the hydrophilic biocompatible polymer (Mn1) to the number average molecular weight of the hydrophobic biocompatible polymer (Mn2) is 1.84 or less [condition (2)]; or
(3) the biocompatible copolymer satisfies all of the above conditions (1) and (2).

2. The powder formulation for tissue repair treatment of Claim 1, wherein the biocompatible copolymer satisfies the condition (1).

3. The powder formulation for tissue repair treatment of Claim 1, wherein the biocompatible copolymer satisfies the condition (2).

4. The powder formulation for tissue repair treatment of Claim 1, wherein the biocompatible copolymer satisfies all of the conditions (1) and (2).

5. The powder formulation for tissue repair treatment of Claim 1, wherein the hydrophilic biocompatible polymer is selected from the group consisting of polyethylene glycol or its derivative, polyvinylpyrrolidone, polyvinyl alcohol, polyacrylamide and combination thereof.

6. The powder formulation for tissue repair treatment of Claim 1, wherein the hydrophobic biocompatible polymer is selected from the group consisting of polylactide, polyglycolide, poly(lactic-glycolide), polymandelic acid, polycaprolactone, polydioxan-2-one, polyamino acid, polyorthoester, polyanhydride, polycarbonate and combination thereof.

7. The powder formulation for tissue repair treatment of Claim 1, wherein the hydrophilic biocompatible polymer is selected from the group consisting of polyethylene glycol (PEG), methoxypolyethylene glycol (mPEG) and combination thereof, and the hydrophobic biocompatible polymer is selected from the group consisting of polylactide, polyglycolide, poly(lactic-glycolide) and combination thereof.

8. The powder formulation for tissue repair treatment of Claim 1, wherein the number average molecular weight of the hydrophilic biocompatible polymer by gel permeation chromatography is from 1,000 g/mol to 30,000 g/mol.

9. The powder formulation for tissue repair treatment of Claim 1, wherein the number average molecular weight of the hydrophobic biocompatible polymer by gel permeation chromatography is from 500 g/mol to 30,000 g/mol.

10. The powder formulation for tissue repair treatment of Claim 1, wherein the number average molecular weight of the biocompatible copolymer by gel permeation chromatography is from 2,000 g/mol to 40,000 g/mol.

11. The powder formulation for tissue repair treatment of Claim 1, further comprising freeze-drying aid.

12. The powder formulation for tissue repair treatment of Claim 11, wherein the freeze-drying aid is one or more selected from the group consisting of lactose, maltose, sucrose, trehalose, mannitol, sorbitol, maltitol, xylitol, and lactitol.

13. The powder formulation for tissue repair treatment of Claim 1, further comprising local anesthetic.

14. The powder formulation for tissue repair treatment of Claim 13, wherein the local anesthetic is one or more selected from the group consisting of ambucaine, amolanone, amylocaine, benoxinate, benzocaine, betoxycaine, biphenamine, bupivacaine, butacaine, butamben, butanilicaine, butethamine, butoxycaine, carticaine, chloroprocaine, cocaethylene, cocaine, cyclomethycaine, dibucaine, dimethysoquin, dimethocaine, diperodon, dicyclonine, ecgonidine, ecgonine, ethyl chloride, etidocaine, beta-eucaine, euprocin, fenalcomine, formocaine, hexylcaine, hydroxytetracaine, isobutyl p-aminobenzoate, leucinocaine mesylate, levoxadrol, lidocaine, mepivacaine, meprylcaine, metabutoxycaine, methyl chloride, myrtecaine, naepaine, octacaine, orthocaine, oxethazaine, parethoxycaine, phenacaine, phenol, piperocaine, piridocaine, polidocanol, pramoxine, prilocaine, procaine, propanocaine, proparacaine, propipocaine, propoxycaine, pseudococaine, pyrrocaine, ropivacaine, salicyl alcohol, tetracaine, tolycaine, trimecaine, zolamine, and salts thereof.

15. A method for preparing a powder formulation for tissue repair treatment, the method comprising:
polymerizing monomers for hydrophobic biocompatible polymer in the presence of hydrophilic biocompatible polymer to prepare a biocompatible copolymer which is a copolymer of hydrophilic biocompatible polymer and a hydrophobic biocompatible polymer; and
drying the prepared copolymer; wherein:
(1) the biocompatible copolymer forms nanoparticles in micelle form in an aqueous medium with an average diameter of from greater than 20 nm to 100 nm or less as measured by dynamic light scattering method [condition (1)]; or
(2) in the biocompatible copolymer, the ratio (Mn1/Mn2) of the number average molecular weight of the hydrophilic biocompatible polymer (Mn1) to the number average molecular weight of the hydrophobic biocompatible polymer (Mn2) is 1.84 or less [condition (2)]; or
(3) the biocompatible copolymer satisfies all of the above conditions (1) and (2).

16. The method for preparing a powder formulation for tissue repair treatment of Claim 15, wherein the monomer for hydrophobic biocompatible polymer is selected from the group consisting of lactide, glycolide, mandelic acid, caprolactone and combination thereof.

17. An injection composition for tissue repair treatment, comprising:
the powder formulation for tissue repair treatment of any one of Claims 1 to 14; and
a pharmaceutically acceptable carrier for injection.

18. A method for preparing an injection composition for tissue repair treatment, the method comprising: mixing the powder formulation for tissue repair treatment of any one of Claims 1 to 14 and a pharmaceutically acceptable carrier for injection at room temperature.
